(19)

**Europäisches Patentamt**
**European Patent Office**
**Office européen des brevets**

(11) **EP 4 078 600 B1**

(12) **EUROPEAN PATENT SPECIFICATION**

(45) Date of publication and mention
of the grant of the patent:
**24.04.2024  Bulletin 2024/17**

(21) Application number: **21702178.1**

(22) Date of filing: **19.01.2021**

(51) International Patent Classification (IPC):
*G16C 20/20* (2019.01)    *H01J 49/00* (2006.01)
*G01N 30/86* (2006.01)

(52) Cooperative Patent Classification (CPC):
**H01J 49/0036; G16C 20/20;** G01N 30/8682

(86) International application number:
**PCT/EP2021/051000**

(87) International publication number:
**WO 2021/148371 (29.07.2021 Gazette 2021/30)**

(54) **METHOD AND SYSTEM FOR THE IDENTIFICATION OF COMPOUNDS IN COMPLEX BIOLOGICAL OR ENVIRONMENTAL SAMPLES**

VERFAHREN UND SYSTEM ZUM IDENTIFIZIEREN VON VERBINDUNGEN IN KOMPLEXEN BIOLOGISCHEN ODER UMWELTPROBEN

PROCÉDÉ ET SYSTÈME POUR L'IDENTIFICATION DE COMPOSÉS DANS DES ÉCHANTILLONS BIOLOGIQUES OU ENVIRONNEMENTAUX COMPLEXES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.01.2020  ES 202030061**

(43) Date of publication of application:
**26.10.2022  Bulletin 2022/43**

(73) Proprietors:
• **Consorcio Centro de Investigación Biomédica en Red**
  **28029 Madrid (ES)**
• **Fundacio Institut d'Investigació Sanitària Pere Virgili**
  **43003 Tarragona (ES)**
• **Universitat Rovira I Virgili**
  **43003 Tarragona (ES)**
• **Giné Bertomeu, Roger**
  **43894 Tarragona (ES)**

(72) Inventors:
• **YANES TORRADO, Óscar**
  **43204 Reus (ES)**
• **CAPELLADES TOMÁS, Jordi**
  **43204 Reus (ES)**
• **GINÉ BERTOMEU, Roger**
  **43894 Tarragona (ES)**

(74) Representative: **Clarke, Modet y Cía., S.L.**
**C/ Suero de Quiñones 34-36**
**28002 Madrid (ES)**

(56) References cited:
**ES-A1- 2 767 375     US-A1- 2017 047 209**

• **ALEXANDRA ZERCK ET AL: "Optimal precursor ion selection for LC-MALDI MS/MS", BMC BIOINFORMATICS, BIOMED CENTRAL , LONDON, GB, vol. 14, no. 1, 18 February 2013 (2013-02-18), page 56, XP021147803, ISSN: 1471-2105, DOI: 10.1186/1471-2105-14-56**
• **SCHMIDT ALEXANDER ET AL: "An Integrated, Directed Mass Spectrometric Approach for In-depth Characterization of Complex Peptide Mixtures", MOLECULAR & CELLULAR PROTEOMICS, vol. 7, no. 11, 15 October 2007 (2007-10-15), pages 2138-2150, XP055795945, US ISSN: 1535-9476, DOI: 10.1074/mcp.M700498-MCP200 Retrieved from the Internet: URL:https://www.ncbi.nlm.nih.gov/pmc/articles/PMC2577211/pdf/3215.pdf>**

EP 4 078 600 B1

**(Cont. next page)**

- NASH WILLIAM J ET AL: "From mass to metabolite in human untargeted metabolomics: Recent advances in annotation of metabolites applying liquid chromatography-mass spectrometry data", TRAC TRENDS IN ANALYTICAL CHEMISTRY, ELSEVIER, AMSTERDAM, NL, vol. 120, 29 November 2018 (2018-11-29), XP085897999, ISSN: 0165-9936, DOI: 10.1016/J.TRAC.2018.11.022 [retrieved on 2018-11-29]
- WANG YANG ET AL: "Enhanced MS/MS coverage for metabolite identification in LC-MS-based untargeted metabolomics by target-directed data dependent acquisition with time-staggered precursor ion list", ANALYTICA CHIMICA ACTA, vol. 992, 13 July 2017 (2017-07-13), pages 67-75, XP085235092, ISSN: 0003-2670, DOI: 10.1016/J.ACA.2017.08.044
- DRAPER JOHN ET AL: "Metabolite signal identification in accurate mass metabolomics data with MZedDB, an interactive m/z annotation tool utilising predicted ionisation behaviour 'rules'", BMC BIOINFORMATICS, BIOMED CENTRAL , LONDON, GB, vol. 10, no. 1, 21 July 2009 (2009-07-21), page 227, XP021055673, ISSN: 1471-2105, DOI: 10.1186/1471-2105-10-227

**Description**

**Field of the invention**

The present invention falls within the field of metabolomics, which is characterised by the analysis of metabolites and small organic molecules in complex biological or environmental samples, such as plasma, urine, tissues and wastewater.

**Background of the invention**

Modern mass spectrometers with very high resolution (> 40,000 FWHM) and mass accuracy (<1-5 ppm), called HRMS (acronym for "High Resolution Mass Spectrometry"), perform mass scans (in MS1 or "full scan" mode) very quickly (in a few milliseconds) in order to analyse ions created by ionised compounds in a complex biological or environmental sample.

When a high resolution mass spectrometer is coupled with a separation technique (known as "hyphenated MS"), for example with liquid chromatography (LC-HRMS, "Liquid Chromatography-High Resolution Mass Spectrometry") or capillary electrophoresis (CE-HRMS, "Capillary Electrophoresis - High Resolution Mass Spectrometry"), for untargeted metabolomic experiments, the raw data matrix can contain tens or hundreds of thousands of data points (also known as scans) in the case of complex samples.

However, until now, the annotation and identification of metabolites by means of LC-HRMS or CE-HRMS in untargeted metabolomic studies is complicated, and the number of metabolites identified is quite limited. The present invention proposes a new method which enables many more (and even all) of the ionised compounds in the biological sample to be identified; therefore, the coverage of possible detected biomarkers increases.

The following documents disclose methods of analysing samples including the creation of inclusion lists for mass spectrometry: Alexandra Zerck et al, "Optimal precursor ion selection for LC-MALDI MS/MS", BMC Bioinformatics, February 2013; Schmidt Alecander et al, "An integrated, directed mass spectrometric approach for in-depth characterization of complex peptide mixtures", Molecular & Cellular Proteomics, October 2007; Nash William et al, "From mass to metabolite in human untargeted metabolomics: Recent advances in annotation of metabolites applying liquid chromatography-mass spectrometry data", Trac Trends in Analytical Chemistry, November 2018; US2017/047209-A1.

**Description of the invention**

The present invention relates to a method for the analysis of untargeted metabolomic data based on coupling mass spectrometry with a separation technique, for example liquid chromatography (LC-MS) or capillary electrophoresis (CE-MS). In mass spectrometry, different ionisation methods can be used to produce ions, such as electrospray ionisation (ESI) or atmospheric pressure chemical ionisation (APCI). The analysed sample can be a biological sample (plasma, tissues, etc.) or a complex environmental sample (i.e., sewage).

According to a first aspect of the present invention, a method for the identification of compounds in complex biological or environmental samples is presented. The method comprises the following steps:

- Receiving raw mass spectrum data from a mass spectrometry analysis coupled with a separation technique applied to a sample, wherein the raw mass spectrum data comprises a plurality of data points with information on retention time, mass-to-charge ratio measured and intensity of the signal measured.
- Consulting a molecular formula database which includes the theoretical mass-to-charge ratio of the molecular ion of a plurality of molecular formulas and ionisation adducts.
- For each data point of the raw mass spectrum data, annotating in an annotation database the combinations of molecular formulas and ionisation adducts the theoretical mass-to-charge ratio of which corresponds to the mass-to-charge ratio measured of said data point considering a given mass error, wherein each annotation includes the retention time and the intensity of the measured signal of the data point.
- For each molecular formula and ionisation adduct annotated in the annotation database, detecting regions of interest defined in a retention time range wherein the annotated data points meet characterisation criteria. Detecting the regions of interest comprises determining candidate regions, defined in a retention time range with a minimum number of data points and/or a minimum density of annotated data points; characterising the candidate regions (20), obtaining characterisation parameters; and selecting as regions of interest those candidate regions the characterisation parameters of which meet certain characterisation criteria.
- Generating an inclusion list which includes the retention time ranges of the detected regions of interest and the theoretical mass-to-charge ratios of the molecular formulas and ionisation adducts associated with each of the regions of interest.

- Sending the inclusion list to a mass spectrometer for the identification of compounds in the sample by means of tandem mass spectrometry.
- Performing a tandem mass spectrometry analysis using the information included in the inclusion list in order to identify compounds in the sample.

The method may comprise a step of detecting in the raw mass spectrum data isotopologues associated with the molecular formulas and/or ionisation adducts annotated. Detecting isotopologues comprises:

- Searching, in the retention time range of each region of interest, for data points of the raw mass spectrum data the mass-to-charge ratio measured of which corresponds, considering a mass error, to a theoretical mass-to-charge ratio of an isotopologue of the molecular formula and/or ionisation adduct associated with the region of interest.
- Obtaining the intensity of the measured signal of the data points found.
- Calculating a theoretical intensity of the data points found starting from the intensity of the measured signal of the data points of the region of interest corresponding to the molecular formula and/or ionisation adduct.
- Comparing the measured intensities with the calculated theoretical intensities.
- Determining the detection of the isotopologue based on said comparison.

The characterisation criteria used can be very diverse:

- Calculating a slope of a linear regression from the data points annotated in the candidate regions, and verifying that the absolute value of the calculated slope is greater than a threshold slope.
- Calculating an average intensity and/or a maximum intensity of the measured signal from the data points annotated in the candidate regions, and verifying that the average intensity and/or the maximum intensity calculated is greater than an average intensity and/or maximum threshold.
- Calculating an intensity range of the measured signal from the data points annotated in the candidate regions, the intensity range being defined by a ratio between the maximum intensity and the minimum intensity in the candidate region, and verifying that the calculated intensity range is greater than a threshold intensity range.
- Calculating a signal-to-noise ratio between an intensity level associated with the data points annotated in the candidate region and an intensity level associated with the data points of the raw mass spectrum data located in an area surrounding the candidate region, and verifying that the calculated signal-to-noise ratio is greater than a threshold signal-to-noise ratio. The area surrounding the candidate region can be defined by a space delimited by:

    i. a mass-to-charge ratio range which includes a mass-to-charge ratio range corresponding to the candidate region, and
    ii. a retention time range which includes the retention time range corresponding to the candidate region.

The method may comprise defining a set of molecular formulas depending on the sample to be analysed, defining ionisation adducts associated with the molecular formulas, and generating the molecular formula database including, for each molecular formula and associated ionisation adduct, the theoretical mass-to-charge ratio.

The method may comprise performing a mass spectrometry analysis coupled with a separation technique applied to the sample in order to obtain the raw mass spectrum data.

A second aspect of the present invention relates to a system for the identification of compounds in complex biological or environmental samples. The system comprises a control unit with data processing means configured to execute the steps of the previously defined method.

The system may comprise a mass spectrometer responsible for performing a mass spectrometry analysis coupled with a separation technique on the sample in order to obtain the raw mass spectrum data.

The system may comprise a mass spectrometer responsible for performing a tandem mass spectrometry analysis using the information included in the inclusion list in order to identify compounds in the sample.

The present invention also relates to a programme product for the identification of compounds in complex biological or environmental samples. The programme product comprises programme instructions for carrying out the previously defined method when the programme is executed in a processor. The programme product may comprise at least one computer-readable storage medium which stores the programme instructions.

**Brief description of the drawings**

What follows is a very brief description of a series of drawings that aid in better understanding the invention, and which are expressly related to an embodiment of said invention that are presented by way of a non-limiting example of the same.

Figure 1 represents a mass spectrum acquired by a mass spectrometer coupled with a separation technique.

Figures 2A and 2B illustrate, according to the state of the art, the detection of region of interest and spectral peaks in the annotation process of the mass spectrum.

Figures 3A, 3B and 3C represent, according to the state of the art, the grouping of spectral peaks in the annotation process of the mass spectrum.

Figures 4A and 4B represent, respectively, the elution forms of the adenosine triphosphate and S-adenosyl methionine.

Figure 5 represents a flow chart of an embodiment of the method of the present invention.

Figures 6A and 6B represent the number of overlaps of formulas and adducts annotated for one same data point of the mass spectrum considering a mass error of 1 ppm and 5 ppm, respectively, of the mass spectrometer.

Figure 7 illustrates a flow chart of the process of detecting regions of interest according to one embodiment.

Figure 8 shows an example of determining and characterising candidate regions.

Figure 9 represents the characterisation of the candidate regions according to different criteria.

Figure 10 illustrates the area surrounding the candidate region used to determine a characterisation criterion of the candidate regions (signal-to-noise ratio).

Figure 11 illustrates a flow chart of the process of detecting isotopologues according to an embodiment of the present invention.

Figures 12A and 12B show two examples of the effects of the resolution of the mass spectrometer on the possible detection of isotopologues.

Figure 13 represents an example wherein the pattern of real and theoretical isotopologues (M1, M2) of a concrete formula (M0) is seen.

## Detailed description of the invention

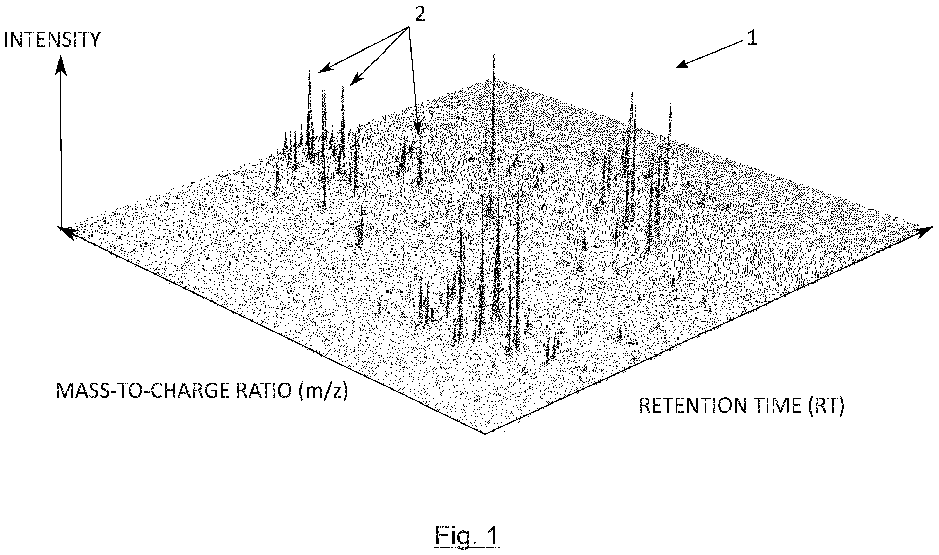

The data points 2 of a mass spectrum 1 acquired in MS1 mode in a mass spectrometer coupled with a separation technique (i.e., liquid chromatography coupled with mass spectrometry, LC-MS, or capillary electrophoresis coupled with mass spectrometry, CE-MS) contains, as represented in the graph of **Figure 1**, three axes of information: mass-to-charge ratio of the detected ions (m/z), intensity (proportional to the abundance of the ions detected), and elution time or retention time RT. Each data point 2 (or scan) of the mass spectrum 1 normally contains information in a fairly wide mass-to-charge m/z range (for example, from m/z of 100 to 1,000) for a given instant in time, being able to have up to thousands of measurements (depending on the resolution of the equipment) of mass-to-charge ratios m/z.

Today, the annotation of the mass spectrum 1 in MS1 mode (MS1 annotation) follows the following scheme:

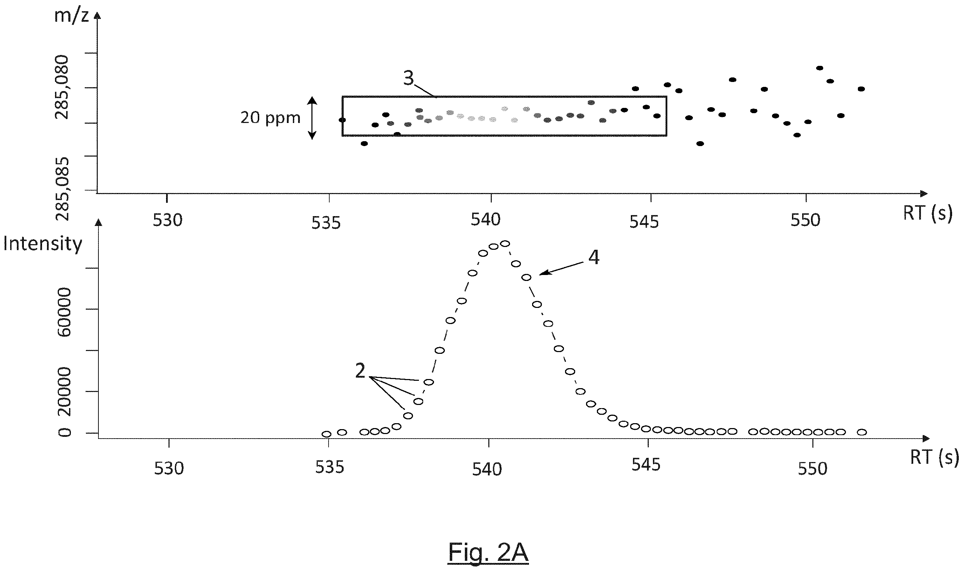

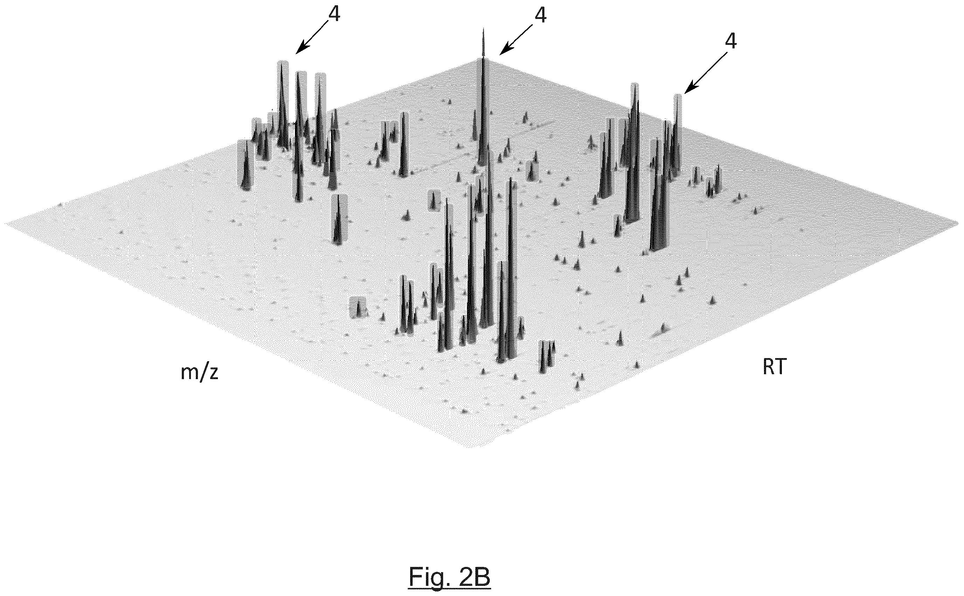

1) An algorithm is used (i.e., CentWave) for the detection of regions of interest 3 (ROI) in the raw data which applies a continuous wavelet transformation and the Gaussian adjustment in the chromatographic separation domain, or any other separation technique coupled with HRMS (on the horizontal axis the retention time RT and on the vertical axis the intensity of the signal measured, as represented in **Figure 2A),** in order to detect spectral peaks 4 throughout the entire mass spectrum 1, for different mass-to-charge ratio (m/z) and retention time (RT) values, as shown in **Figure 2B.**

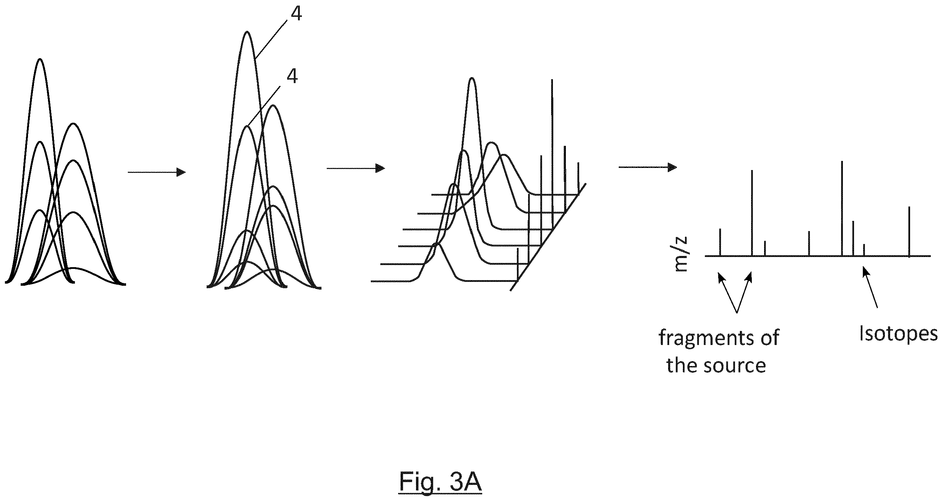

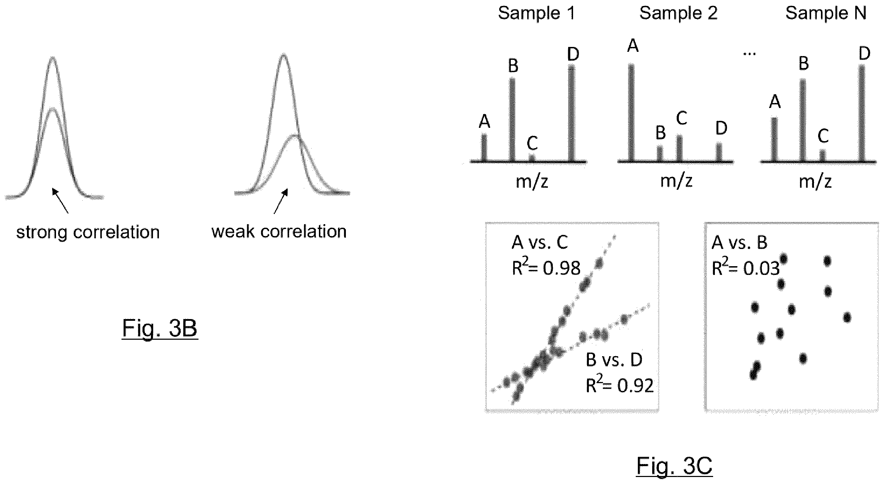

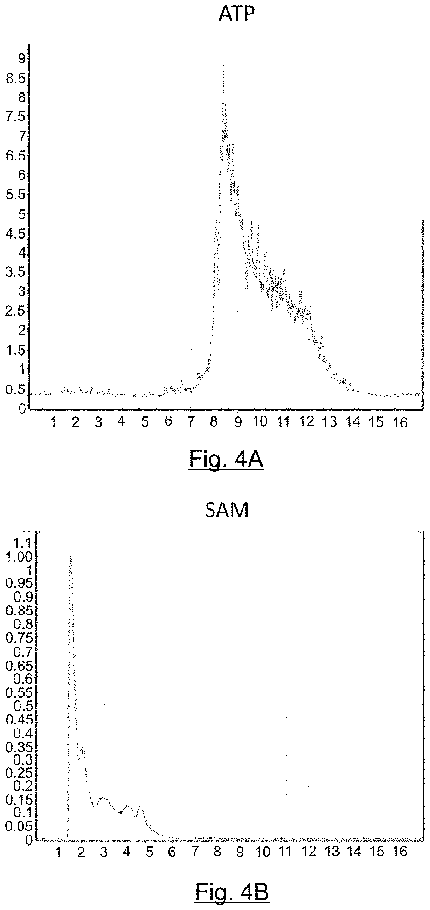

2) Subsequently, another algorithm (i.e., CAMERA, CliqueMS) groups the spectral peaks 4 belonging to the same compound due to the redundancy that exists for adducts and isotopes (**Figure 3A**). The fragments of the source shown in Figure 3A are mainly due to the loss of a water, i.e.: [M-H2O+H]+ in positive ionisation, or [M-H2O-H]- in negative ionisation. The grouping of spectral peaks 4 can be performed by means of a correlation of the shape of the peak (**Figure 3B),** wherein a high correlation of the shape of the peaks is sought. Spectral peaks 4 showing a weak correlation are not grouped. The grouping can also be performed by means of a correlation of the abundance or intensity of the peaks using different samples. In the example of **Figure 3C**, an almost constant ratio is observed between the intensity of the peak of the mass-to-charge ratio A and the intensity of the peak of the mass-to-charge ratio C (coefficient of determination of the linear regression $R^2 = 0.98$). Similarly, there is a strong correlation between the mass-to-charge ratio B and the mass-to-charge ratio D ($R^2 = 0.92$). However, there is no correlation between the mass-to-charge ratios A and B ($R^2 = 0.03$). However, this method has serious limitations when the elution forms of the metabolites do not fit the function (i.e., Gaussian) which is intended to fit the data, which occurs for example with adenosine triphosphate (ATP, **Figure 4A**) or S-adenosyl methionine (SAM, **Figure 4B**).

After completing the MS1 annotation, for the characterisation or identification of metabolites an MS2 annotation is performed by using tandem mass spectrometry or $MS^n$ ($n \geq 2$). There are currently three methods for the identification of metabolites by means of LC-MS/CE-MS (or LC-HRMS/CE-HRMS) and $MS^n$ in untargeted metabolomics:

- Inclusion list (targeted MS/MS): Samples are analysed in $MS^1$ mode and the data is processed by means of one or more software programmes which detect and align peaks (as explained in Figure 3A). Normally following criteria of statistical changes between groups or experimental conditions, a proportion of these m/z are fragmented by means of $MS^2$ or $MS^n$ analysis in a subsequent experiment.

- Data-dependent acquisition (DDA): The mass spectrometer collects $MS^1$ and $MS^n$ data in the same untargeted metabolomic analysis. A short work cycle of $MS^1$ recognition of the m/z eluting at that moment serves to control the intensity of the m/z and to identify/select possible m/z to fragment. Then, "n" $MS^2$ or $MS^n$ cycles are applied, during each of which a single m/z precursor is isolated and fragmented, and the fragments thereof are detected. The precursors are fragmented in the order of decreasing intensity. A dynamic exclusion window is typically used to ensure that the m/z that have been recently scanned by $MS^2$ do not constantly re-fragment if new m/z are available.

- Data-independent acquisition (DIA): The mass spectrometer collects $MS^1$ and $MS^n$ data in the same untargeted metabolomic analysis. It is a method of determining molecular structures wherein all the ions within a selected m/z range are fragmented and the mixture of fragments is detected. The mass spectra in $MS^n$ are acquired either by fragmenting all the ions entering the mass spectrometer at a given time (broadband DIA) or by isolating and sequentially fragmenting all the ions in m/z ranges (SWATH™, Sequential windowed acquisition of all theoretical mass spectra).

The present invention consists of a new method for processing raw data from an LC-HRMS or CE-HRMS analysis in MS1 mode and selecting mass-to-charge ratios (m/z) and retention time (RT) ranges for the identification of metabolites in a subsequent analysis performed by means of tandem mass spectrometry or $MS^n$ (n≥2).

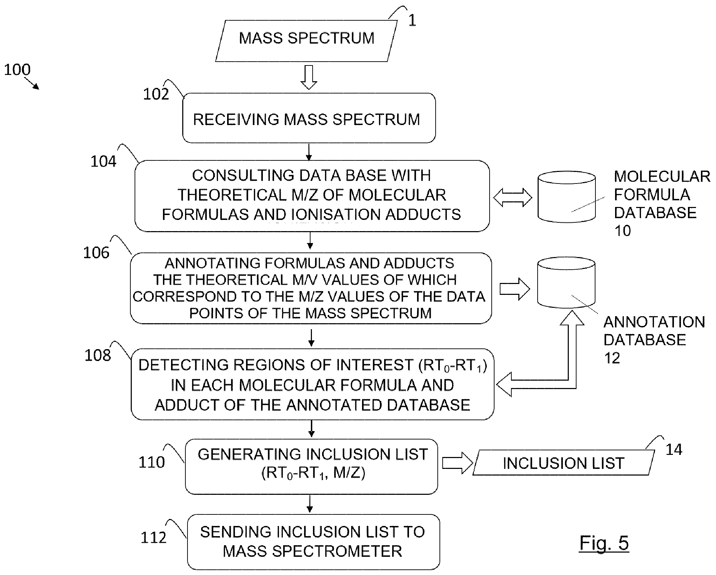

The method 100 of the present invention comprises the steps shown in the flow chart of **Figure 5.**

First, the method 100 comprises receiving 102 a mass spectrum 1 from an LC-MS or CE-MS analysis (acquired in MS1 mode) applied on a biological or environmental sample. The mass spectrum 1 comprises a plurality of data points 2 with information including the retention time (RT), the mass-to-charge ratio (m/z) measured and the intensity of the signal measured.

Next, a molecular formula database 10 is accessed or consulted 104 including the theoretical mass-to-charge ratio $(m/z)^T$ of the molecular ion of a plurality of molecular formulas and associated ionisation adducts. In one embodiment, the molecular formula database 10 comprises a list of formulas and a list of adducts, with the theoretical charge-to-mass ratio of each formula and each adduct, such that the theoretical charge-to-mass ratio of the combinations of molecular formulas and ionisation adducts can subsequently be calculated starting from the monoisotopic mass of the molecular formula plus the difference in mass that the ionisation adduct contributes when charged at the source (i.e., H, Na K). In another embodiment, the molecular formula database 10 directly stores the theoretical charge-to-mass ratio of the different combinations of formulas and adducts, such that no subsequent calculation is necessary.

The contents of the molecular formula database 10, or the information accessed in the consultation 104, are preferably oriented towards the particular sample to be analysed, based on a large universe or space of molecular formulas related to the matrix to be analysed (serum, urine, cells, environmental samples, etc.). In the case of biological matrices of biomedical interest, the molecular formulas included in the Human Metabolome Database (HMDB) can be used. For example, a set of molecular formulas can be defined depending on the sample to be analysed and ionisation the association of which with the molecular formulas is known. Databases can be considered which include only the molecular formulas oriented towards the particular sample (for example with the formulas which are expected to be found in blood plasma), or larger databases, like the HMDB database which includes information on more than 10,000 metabolites found in the human body.

Once the molecular formulas and the ionisation adducts thereof are defined, the contents of the molecular formula database 10 can be generated including, for each molecular ion of the molecular formula and for each associated ionisation adduct, the theoretical mass-to-charge ratio $(m/z)^T$, which can be obtained directly from the molecular formula considering the corresponding atomic weights. The method may comprise the step of generating the molecular formula database 10. Alternatively, the molecular formula database 10 may have already been created prior to the implementation of method 100, such that the method 100 only requires accessing a memory (i.e., on a local device or in the cloud) wherein the previously generated molecular formula database 10 is stored.

The construction of the molecular formula database 10 may comprise the generation of a table containing all the theoretical mass-to-charge ratios $(m/z)^T$ after considering the main isotopologues (i.e., M1, M2, M3) and known adducts in both positive and negative ionisation (the fragments at the source can be considered as an adduct in the adduct list) for each unique molecular formula considered. The information contained in the molecular formula database 10 can, for example, be structured in the form of a table, wherein a different formula/adduct/isotopologue is included in each row. The table can be ordered by theoretical mass-to-charge ratio $(m/z)^T$, the first column, as represented in the following example:

| m/zT | Formula | Adduct | Isotopologue |
|---|---|---|---|
| 376.2312 | C21H27NO4 | +NH4 | M1 |

The method searches for all the theoretical mass-to-charge ratio $(m/z)^T$ values at each data point 2 of the LC-MS or CE-MS mass spectrum 1, within a predefined error (typically 1 to 5 ppm). Alternatively, a scan is made at the data points 2 of the mass spectrum 1 and it is verified, for each data point 2, whether the mass-to-charge ratio (m/z) thereof measured corresponds to a theoretical mass-to-charge ratio $(m/z)^T$ from the molecular formula database 10. In order to facilitate the search, the molecular formula database 10 can include the data ordered from lowest to highest theoretical mass-to-charge ratio $(m/z)^T$.

For each data point 2 of the mass spectrum 1, the molecular formulas and ionisation adducts the theoretical mass-to-charge ratio $(m/z)^T$ of which corresponds to the measured mass-to-charge ratio (m/z) of said data point are annotated 106 in an annotation database 12, considering a certain margin or mass error (coming from the accuracy of the measuring or calibration of the mass spectrometer). The annotation database 12 includes, for each molecular formula and ionisation adduct annotated, the retention time (RT) and the intensity of the signal measured of the data point associated with the formula/adduct. The information contained in the annotation database 12 can be structured for example in the form of a table, wherein a different annotation is included in each row. Each row will therefore be a new annotation which will include the formula and/or adduct annotated, the corresponding retention time (RT) thereof, the intensity of the signal measured of the data point 2 of the associated mass spectrum 1 and, optionally, the mass-to-charge ratio (m/z) measured.

| Formula | Adduct | RT | Intensity | m/z measured |
|---|---|---|---|---|
| C21H27NO4 | +NH4 | | | 375.2281 |

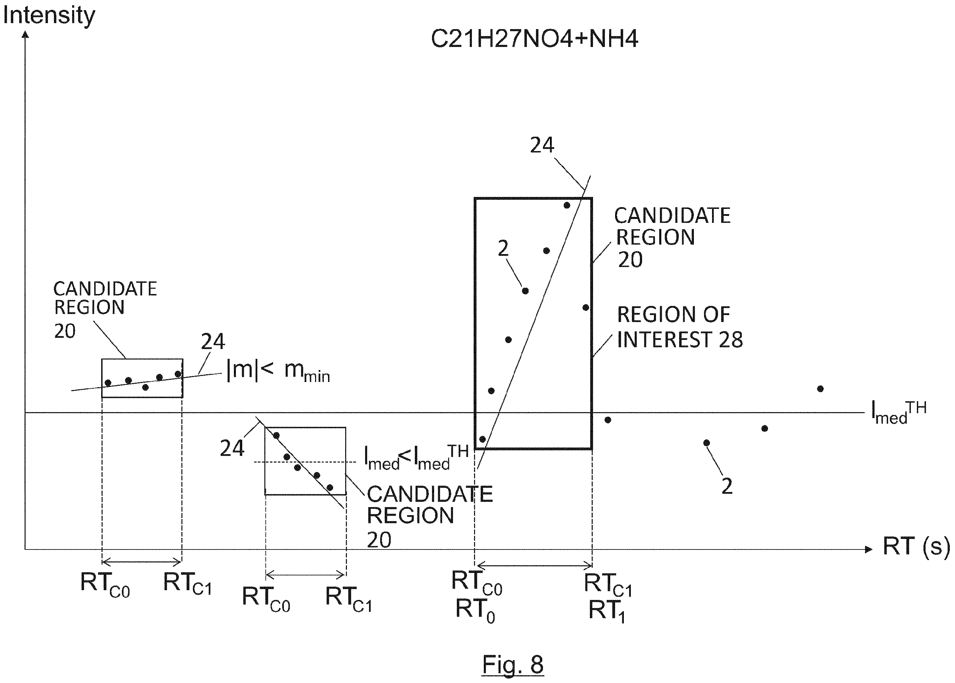

The different annotations of retention times (RT) and intensity which are made in the annotation database 12 for one same formula and adduct in different rows of the table can be grouped (and even represented in a graph, as shown in Figure 8 for the adduct +NH4 of the formula C21H27NO4) for the subsequent analysis detecting regions of interest.

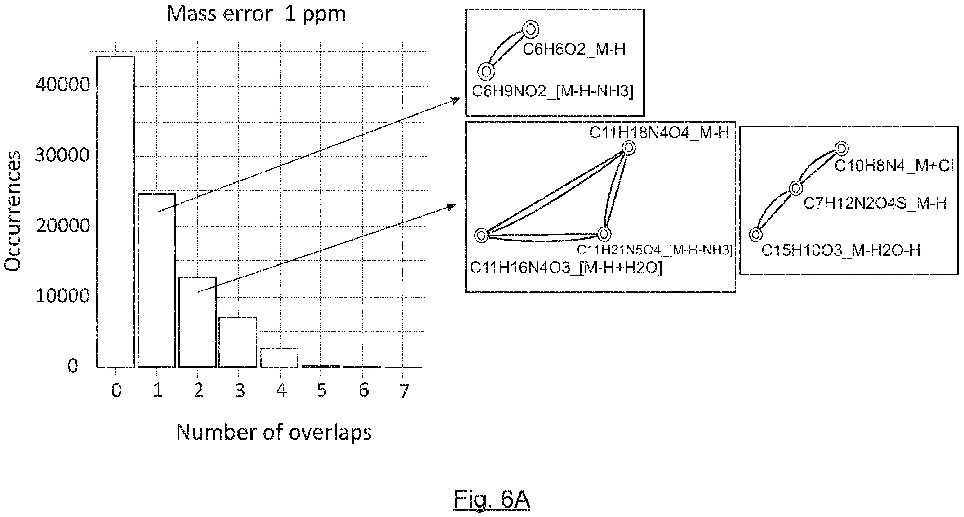

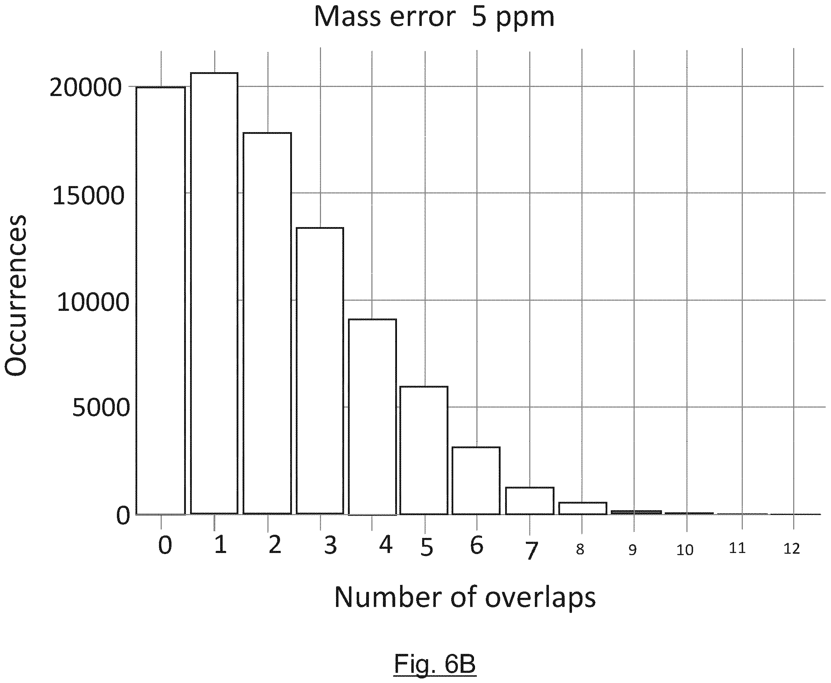

According to the mass error defined, there will be more or less overlap of possible formulas and/or adducts annotated for one same data point 2. In the graph of **Figure 6A,** the number of overlaps produced is represented on the horizontal axis, considering a mass error of 1 ppm, for the different mass-to-charge ratios (m/z) of the data points of the mass spectrum 1 (from 0 overlaps to 7 overlaps), and on the vertical axis, the number of occurrences for each different overlap number. For example, the mass-to-charge ratio (m/z) measured at a data point 2 of the mass spectrum 1 can correspond to two different formulas/adducts, considering the mass error of 1 ppm: the negative ionisation adduct -H-NH3 of the molecular formula C6H9NO2, and the negative molecular ion -H of the molecular formula C6H6O2. There is therefore an overlap between two formulas and/or adducts. When the overlap occurs between N formulas and/or adducts, N-1 overlaps are considered to exist. Of the nearly 100,000 data points 2 of the mass spectrum 1 in the example of Figure 6A, at more than 40,000 data points there are no overlaps, at more than 20,000 data points there is 1 overlap and at more than 10,000 data points there are two overlaps. As the mass error increases, the overlap between different possible formulas-adducts increases (in **Figure 6B** the overlap with 5 ppm mass error is shown).

Next, once the annotation 106 has been performed, each molecular formula and ionisation adduct of the annotation database 12 is then analysed, grouping all the annotations that occurred for one same formula/adduct (see example of Figure 8), in order to detect 108 regions of interest defined in a retention time range $(RT_0-RT_1)$ wherein the data points annotated meet certain characterisation criteria. A single formula/adduct from the annotation database 12 may include a single region of interest or several regions of interest detected in different retention time ranges.

The method 100 implements an algorithm in order to find regions of interest based on verifying one or more characterisation criteria, first considering a criterion of minimum density and/or minimum number of data points in the region of interest (which will determine candidate regions), and considering additional criteria afterwards, such as a minimum slope of the data points in the region of interest or a certain minimum signal-to-noise ratio. The detected regions of interest can also be compared, in an optional but recommended manner, with a sample blank in order to rule out false positives or data points exogenous to the sample.

Therefore, and unlike the state of the art, determining the regions of interest does not consist of finding peaks in the mass spectrum 1 by fitting a model (i.e., Gaussian) to the data. The approach of the new method is independent from the shape and determination of the spectral peaks 4, it not being necessary to make any type of correlation between the spectral peaks (as shown in Figures 3B and 3C for the state of the art). This makes the method of the present invention independent from chromatographic conditions.

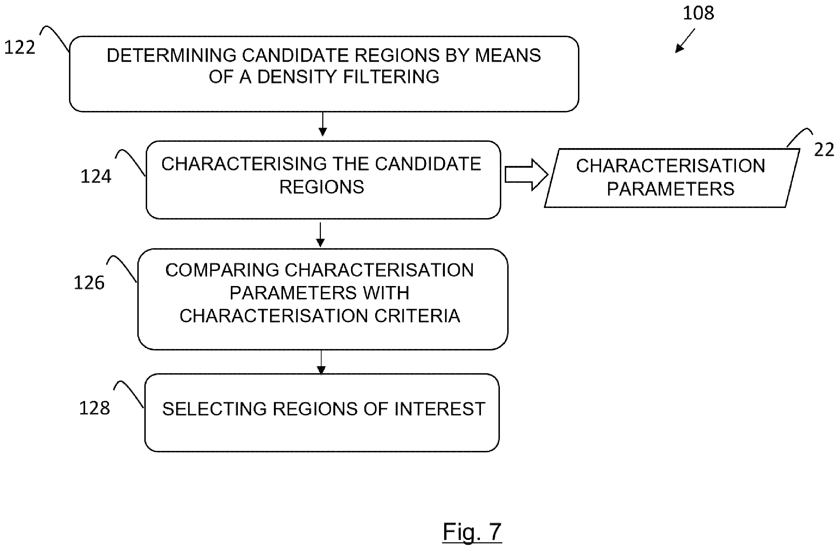

**Figure 7** shows a flow chart of the process of detecting 108 regions of interest according to one embodiment. Detecting 108 regions of interest comprises determining 122, for each molecular formula and ionisation adduct of annotation database 12, candidate regions 20 defined in a retention time range ($RT_{C0}$-$RT_{C1}$), like the ones represented in the example of **Figure 8**, with a minimum number of data points and/or a minimum density of data points annotated in the candidate region 20. This step corresponds to filtering by density of data points, only considering possible regions of interest (i.e., candidate regions 20) for those time windows which group a minimum number of data points and/or a minimum density of data points. In the example shown in Figure 8, the data points 2 of the mass spectrum 1 annotated in the annotation database 12 are represented for the adduct $[M+NH4]^+$ of the molecular formula C21H27NO4. Candidate regions 20 that have passed the density filtering are also shown; for example, the time ranges containing at least five data points 2 of the mass spectrum 1 in a certain maximum range of time are selected as candidate regions 20.

Next, the candidate regions 20 are characterised 124, obtaining characterisation parameters 22 of the candidate regions 20. Lastly, the characterisation parameters 22 obtained are compared 126 with characterisation criteria, and those candidate regions 20 the characterisation parameters 22 of which meet certain characterisation criteria are selected 128 as regions of interest.

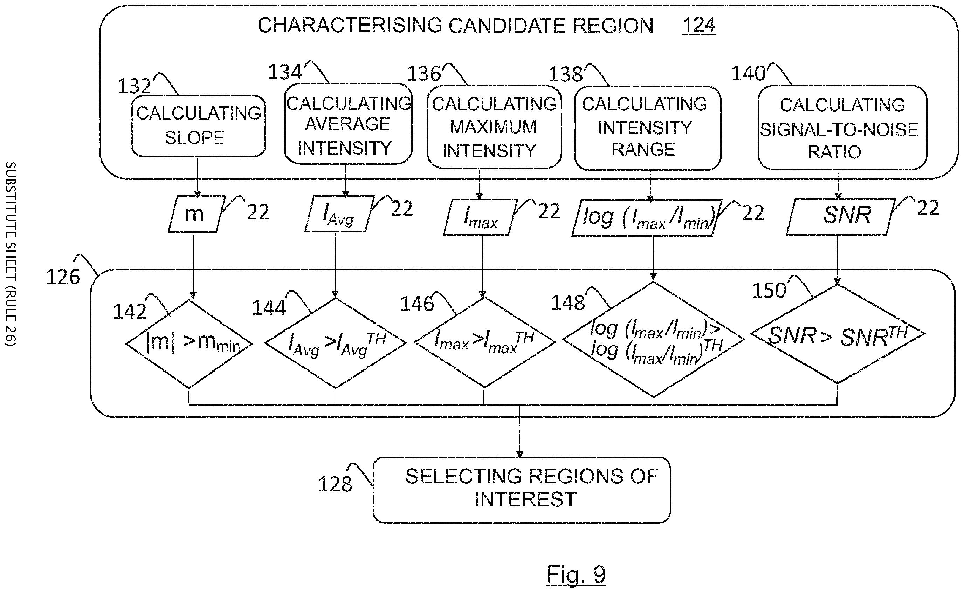

**Figure 9** shows different manners of characterising 124 the candidate regions 20 and different characterisation criteria which it can be considered that the candidate regions 20 must fulfil. For example, considering, among others, any combination of the following characterisation criteria:

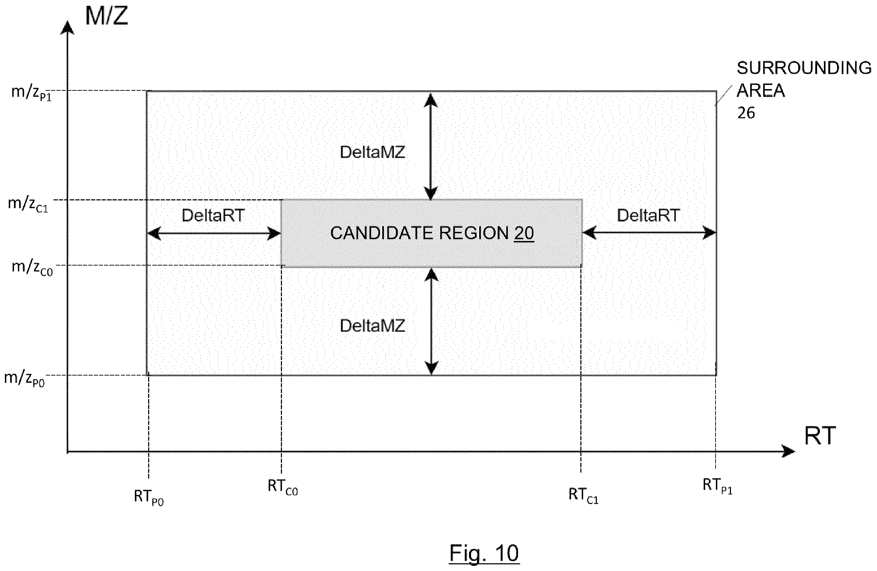

- A minimum slope of the data points 2 in the candidate region 20: The characterisation of the candidate regions 20 may comprise calculating 132 the slope (m) of a linear regression 24 (see Figure 8) of the data points 2 annotated in the candidate regions 20. The characterisation criteria may include verifying 142 that the absolute value of the calculated slope is greater than a minimum slope ($m_{min}$) or threshold slope.
- An average and/or maximum intensity of the signal measured in the candidate region: The characterisation of the candidate regions may comprise calculating 134 an average intensity ($I_{avg}$) and/or calculating 136 a maximum intensity ($I_{max}$) of the measured signal from the data points 2 annotated in the candidate regions 20. The characterisation criteria may include verifying 144 that the average intensity ($I_{avg}$) calculated is greater than a threshold average intensity ($I_{avg}^{TH}$) and/or verifying 146 that the maximum intensity ($I_{max}$) calculated is greater than a maximum threshold intensity ($I_{max}^{TH}$).
- An intensity range in the candidate region: The characterisation of the candidate regions may comprise calculating 138 an intensity range of the measured signal from the data points annotated in the candidate regions, wherein the intensity range is defined by a ratio between the maximum intensity and the minimum intensity in the candidate region (e.g., a logarithmic ratio between the maximum intensity value, $I_{max}$, and the minimum intensity value, $I_{min}$, of the data points 2 annotated in candidate region 20). The characterisation criteria may include verifying 148 that the calculated intensity range is greater than a threshold intensity range.
- A minimum signal-to-noise ratio (SNR): The characterisation of the candidate regions may comprise calculating 140 a signal-to-noise ratio (SNR) between an intensity level associated with the data points 2 annotated in the candidate region 20 and an intensity level associated with the data points 2 of the mass spectrum 1 located in an area surrounding the candidate region 20. The characterisation criteria may include verifying 150 that the signal-to-noise ratio (SNR) calculated is greater than a threshold signal-to-noise ratio ($SNR^{TH}$). According to the embodiment shown in **Figure 10,** the area 26 surrounding the candidate region 20 can be defined by a space delimited by a mass-to-charge ratio range ($m/z_{P0}$-$m/z_{P1}$) which includes the mass-to-charge ratio range ($m/z_{C0}$-$m/z_{C1}$) corresponding to the candidate region 20, and for a retention time range ($RT_{P0}$-$RT_{P1}$) which includes the retention time range ($RT_{C0}$-$RT_{C1}$) corresponding to the candidate region 20, wherein said space may or may not include the candidate region 20 itself (in Figure 10 the range $m/z_{C0}$-$m/z_{C1}$ of the candidate region is not to scale, it has been expanded for illustrative purposes; in practice, the range $m/z_{P0}$-$m/z_{P1}$ is much larger than the range $m/z_{C0}$-$m/z_{C1}$, even up to 100,000 times larger). The candidate region can be considered to include a mass-to-charge ratio range ($m/z_{C0}$-$m/z_{C1}$) since it is considered a mass error in the annotation in the annotation database 12.
- A minimum and/or maximum amplitude of the retention time range ($RT_0$-$RT_1$) of the regions of interest (i.e., minimum and/or maximum distance of time from the beginning to the end of the region ($RT_0$-$RT_1$)).

However, it is possible to use other different characterisation parameters or criteria. Furthermore, the characterisation criteria can be coupled with machine learning techniques (artificial neural networks, random forests, etc.) in order to filter candidate regions 20 and generate a more specific inclusion list in exchange for applying a bias associated with the learning method itself.

In the example of Figure 8, the candidate region on the left is not selected as the region of interest because it does not meet the criterion of a minimum slope ($|m|<m_{min}$). The candidate region in the middle is also not selected as the region of interest because the average intensity ($I_{avg}$) of the data points 2 thereof is less than a threshold average intensity ($I_{avg}^{TH}$). The candidate region 20 on the right is selected 128 as the region of interest 28 because the characterisation parameters 22 meet the required characterisation criteria (i.e., $|m|>m_{min}$; $I_{med} > I_{med}^{TH}$, etc.). In the case represented, the region of interest 28 matches the candidate region ($RT_{CO}=RT_0$, $RT_{C1}=RT_1$). However, the region of interest 28 finally considered may result from the grouping of other overlapping regions (for example, grouping candidate regions or other overlapping regions of interest).

The method 100 continues with the generation 110 of an annotated and highly accurate inclusion list 14, with variable time ranges according to the elution profile of each m/z, for MS/MS (or MS$^n$) experiments which facilitates the identification of metabolites. The inclusion list 14 includes the retention time ranges ($RT_0$-$RT_1$) of the detected regions of interest and the theoretical mass-to-charge ratios $(m/z)^T$ of the molecular formulas and/or ionisation adducts associated with each of the detected regions of interest. Optionally, the inclusion list may also include the molecular formulas and/or ionisation adducts associated with each of the detected regions of interest.

Lastly, the inclusion list 14 is sent 112 to a mass spectrometer in order to, by means of a tandem mass spectrometry analysis, perform the identification of metabolites in the sample by using the data from the inclusion list 14. Optionally, the method may comprise performing the tandem mass spectrometry analysis by using the information comprised in the inclusion list in order to identify metabolites in the sample. The MS/MS analyses are subsequent to the mass scans in MS1 mode performed in the LC-MS analysis, requiring a second injection of the same sample since currently there is no technology for accumulating or storing ions after being detected in the MS1.

The new method analyses the data points of the mass spectrum of a representative biological sample, acquired in MS1 mode, in order to select those mass-to-charge ratios m/z (and the time ranges thereof) which will be fragmented in subsequent MS$^n$ experiments. A novel aspect of the present invention is the manner of selecting the mass-to-charge ratios (m/z) and the retention time ranges in order to perform the MS$^n$ analysis, since it is not based on the detection of peaks, it is a method independent from the chromatographic elution profile of the compound, being able to detect metabolites with non-Gaussian elution shapes or similar (such as those of Figures 4A and 4B). Furthermore, in the event that the molecular formulas and/or ionisation adducts associated with the detected regions of interest are sent to the mass spectrometer, the mass spectrometer can use this information in post-fragmentation analysis in order to more quickly identify the compounds, since it starts from a certain list of candidate formulas.

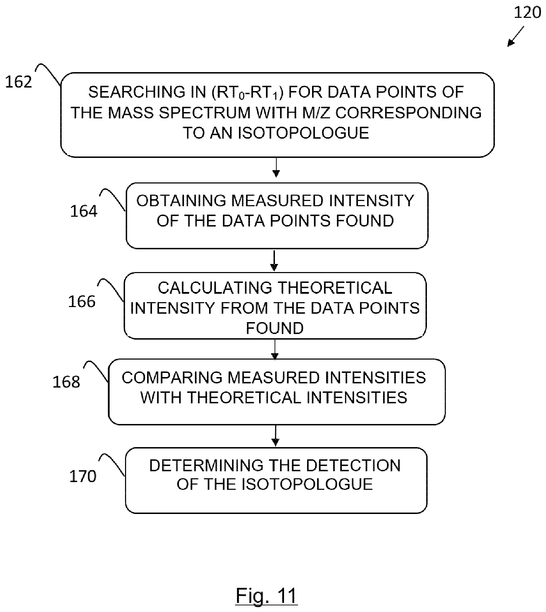

Furthermore, the present invention presents a novel manner of detecting isotopologues of molecular formulas and/or ionisation adducts in the mass spectrum 1. The detection of isotopologues can be verified once the regions of interest 28 of the molecular formulas and ionisation adducts have been detected 108. The detection of isotopologues 120 comprises, as represented in the flow chart of **Figure 11**, searching 162 in the retention time range ($RT_0$-$RT_1$) of each region of interest 28 (or at least in a time interval comprised in said range $RT_0$-$RT_1$) for data points 2 of the mass spectrum 1 the measured mass-to-charge ratio (m/z) of which corresponds, considering a mass error, to a theoretical mass-to-charge ratio $(m/z)^T$ of an isotopologue (i.e., M1) of the molecular formula and/or ionisation adduct (M0) associated with the region of interest. In this manner, it is verified that the mass-to-charge ratio m/z agrees with the theoretical one of the isotopologue, considering a mass error of the spectrometer.

Next, the intensity of the signal measured of each of the data points found in the search 162 is obtained 164. A theoretical intensity of the data points found in the search 162 is calculated 166 starting from the intensity of the data points of the region of interest (i.e., the data points corresponding to the main formula/adduct M0), and depending on the theoretical abundance ratio of the isotopologue in question (whether it be M1, M2, etc.) which is expected to be found with respect to the main formula or adduct M0. For example, if the theoretical abundance ratio of an isotopologue M1 is 2.5 % with respect to the main formula/adduct M0, the theoretical intensity of the isotopologue would be 2.5 % of the intensity level from the data points of the region of interest. the measured intensities are compared 168 with the theoretical calculated intensities and the detection or not of the isotopologue is determined 170 based on this comparison. In one embodiment it is verified, for each of the data points found, if the measured intensity of the data point corresponds to the theoretical intensity of the isotopologue, considering a certain intensity margin (in order to contemplate, for example, possible sensitivity errors in the measuring or divergence with respect to the theoretical abundance ratio of the isotopologue with respect to the formula/adduct M0). In order to calculate the theoretical intensity of the isotopologue, the intensity of the corresponding M0 (*Int(M0)*) (i.e., the intensity of the signal measured of the data point of the region of interest 28 at a corresponding instant in time RT -in the same scan-) and the theoretical abundance ratio (*ratio*) of the isotopologue with respect to the M0. In the comparison 168 of the measured intensities with the theoretical intensities, an intensity margin is considered; for example, verifying that the measured intensity of the isotopologue (*Int(iso)*) is included in a constructed interval (as a function of a value k) around the theoretical value (*Int(M0)\*ratio*) which would correspond to the isotopologue:

$$Int(M0)*ratio*(1+k) > Int(iso) > Int(M0)*ratio*(1-k)$$

Then an additional verification based on cosine similarity comparison can optionally be performed, which is defined as:

$$similarity = \cos(\theta) = \frac{\mathbf{A} \cdot \mathbf{B}}{\|\mathbf{A}\|\|\mathbf{B}\|} = \frac{\sum_{i=1}^{n} A_i B_i}{\sqrt{\sum_{i=1}^{n} A_i^2}\sqrt{\sum_{i=1}^{n} B_i^2}},$$

Said verification can be performed in the following manner:

- The annotation database 12 is searched for the entries corresponding to the conditions to be compared (i.e., M0 compared to an isotopologue M1) in the RT interval corresponding to the region of interest being analysed corresponding to the M0.
- All those entries in each set which share the retention time RT are searched for (in other words, that entries of the two conditions M0 and M1 have been found in one same scan -i.e., same instant in time RT-).
- If there are enough entries (i.e., more than 5, in order to avoid false positives when N is small), cosine similarity is calculated (with I = <i1,i2,i3...iN> and J = <j1,j2,j3...jN>, the vectors of the intensities of the two conditions to be compared):

$$Cos = (i1j1 + i2j2 + ... iNjN) / (module(I)*module(J))$$

- If Cos > k (i.e., k=0.99), then it is determined that an isotopologue has been found and which one it was is recorded.

The search 162 for data points corresponding to an isotopologue with a certain formula and adduct can be performed by consulting the annotation database 12, which can include annotations of the isotopologues (M1, M2,...), in addition to the annotations of the formulas/adducts (M0). To this end, when performing an annotation 106 of a formula/adduct (M0), the existence of a data point with mass-to-charge relation corresponding to an isotopologue (M1, M2,... ) and an intensity close to the theoretical is verified, and in that case the annotation of the isotopologue is performed. Alternatively, the search 162 for isotopologues can be performed directly in the mass spectrum 1 (since the instant in time RT and the mass-to-charge ratio to be searched for are known).

The search for the isotopologues the presence or absence of which must be determined for each formula and/or adduct annotated can be determined in the molecular formula database 10, which may include, for example, the isotopologues to be considered for each formula and/or adduct (for example, the main isotopologues M1 and M2 of each formula/adduct M0) and the corresponding theoretical mass-to-charge ratio $(m/z)^T$ thereof. The molecular formula database 10 may also include the theoretical abundance ratio of the isotopologue. In one embodiment, the isotopologues which can theoretically be detected are determined based on the mass resolution of the spectrum in the mass-to-charge ratio m/z range analysed, this makes it possible to adjust for each M0 the space of isotopologues that the mass spectrometer can detect depending on the resolution of the equipment. The information related to the isotopologues can be included for example in an isotopologue database, wherein the composition of the isotopologues (M1, M2,...) detectable with the mass spectrometer, the mass-to-charge ratio m/z with respect to the M0 and the abundance ratio are stored.

Therefore, the method makes it possible to calculate the isotopic pattern of each formula and to differentiate which isotopologues are detectable by the apparatus given the intensity ratio with respect to the M0 and the resolution of the mass spectrometer. The method to determine if the peaks of the calculated isotopologues are separable depends on the mass analyser used (as explained for example in the document "Orbitrap Mass Spectrometry", Zubarev et al., Analytical Chemistry 2013, 85 (11), pages 5288-5296). In the case of Orbitrap analysers, the resolution is inversely proportional to the square root of the m/z, and therefore it can be calculated mathematically. In the case of FTICR analysers, the resolution has an inverse scale to the m/z, for which reason it can also be calculated mathematically. In contrast, the resolution in TOF analysers is independent from the m/z, for which reason the resolution of each m/z is calculated by means of a calibration curve.

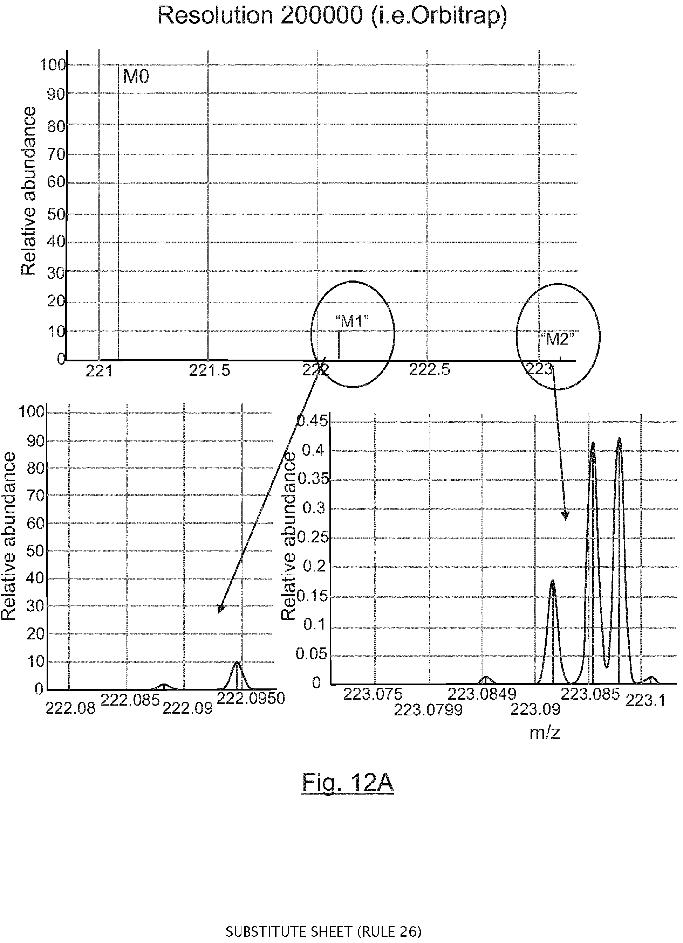

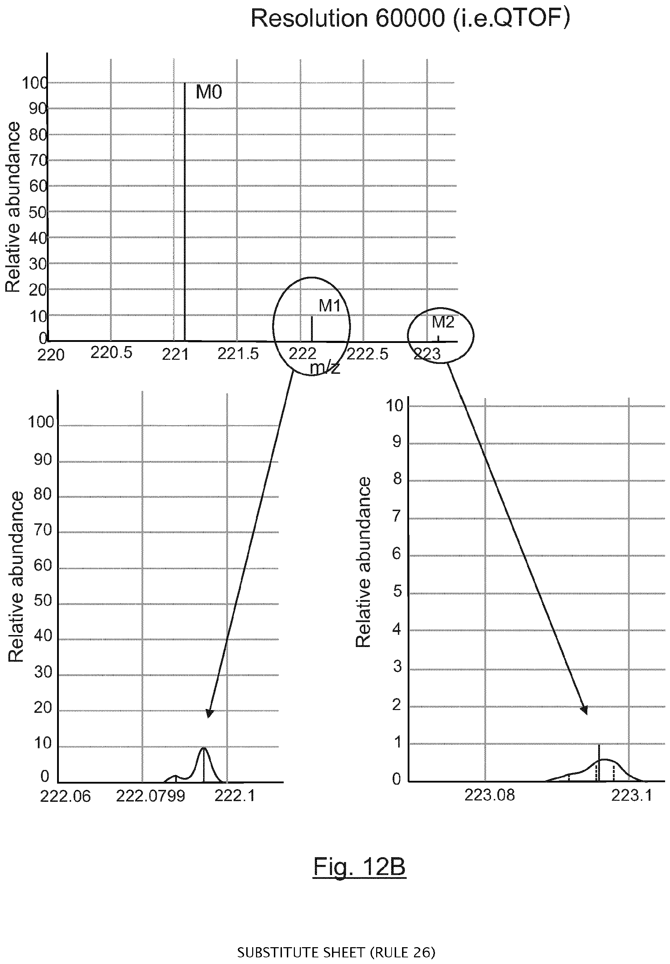

Figures 12A and 12B show an example of isotopic patterns (M0, M1 and M2) of phenylalanine (C9H11N5O2), explaining therein the effect of the resolution in order to distinguish isotopologues and how a higher resolution enables other isotopologues other than M1 and M2 to be distinguished. Figure 12A corresponds to a 200000 resolution (Orbitrap) and Figure 12B to a 60000 resolution (QTOF). These figures show:

- Dashed vertical lines: showing the theoretical mass-to-charge ratios m/z (and the abundance thereof), according to the relative abundance of each natural isotope of each atom.
- Curved lines: As a consequence of the equipment, according to the resolution thereof, they cannot perfectly distinguish between the theoretical mass-to-charge ratios m/z (if appropriate, the ones detected), what is really seen in the mass spectrum is a curve which encompasses them (curved line). According to the resolution, that curve defines the broken vertical lines better or worse.

- Continuous vertical lines: these are a simplification (a sum) of the curved line, a manner of preventing all the data points of the curved line from being collected, and instead encompassing them in a single signal (called a centroid). This value is a "weighted average" of the mass-to-charge ratios m/z encompassed within the curved line, and the abundance thereof.

In the example of Figure 12A, the 200000 resolution (Orbitrap) is sufficient to completely separate the isotopologues M1 and M2. However, for the case shown in Figure 12B (resolution of 60000, QTOF), the isotopologues M1 can be separated but the isotopologues M2 are indistinguishable and cannot be separated.

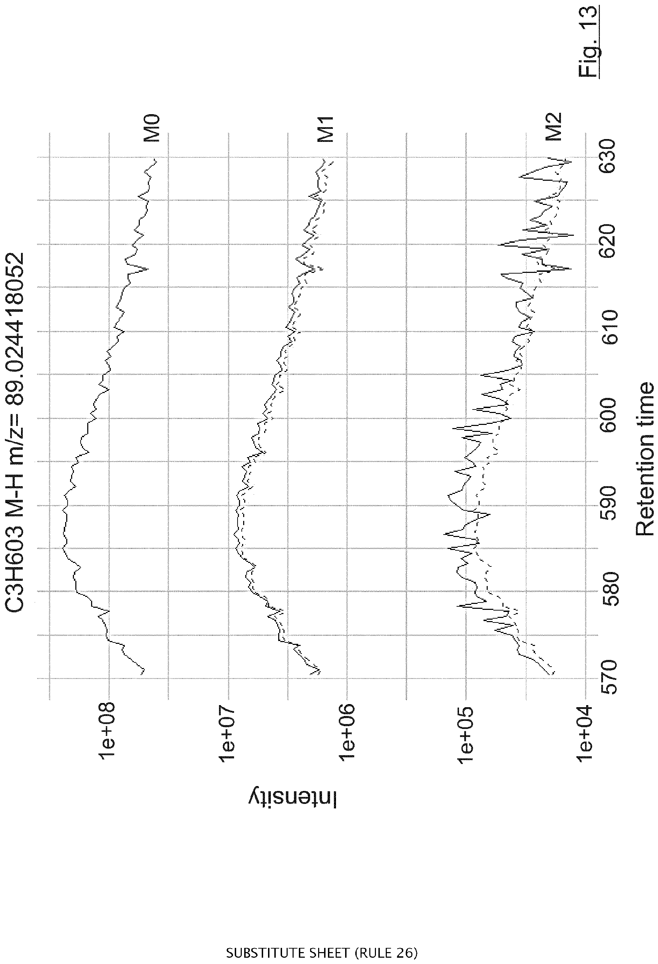

**Figure 13** represents a real example wherein the pattern of isotopologues (M1, M2) of a particular formula M0 is seen, as well as how they approximately follow the calculated theoretical intensity ratio (in dashed line).

In case of overlapping of several formulas-adducts for a given mass-to-charge ratio (m/z), the number of isotopologues associated with one same formula that have been detected by the method can be used to prioritise one candidate formula over another, providing relevant information about which compound can be treated before even performing the tandem mass spectrometry.

## Claims

1. A computer-implemented method for the identification of compounds in complex biological or environmental samples, **characterised in that** it comprises:

   receiving (102) raw mass spectrum data (1) from a mass spectrometry analysis coupled with a separation technique applied to a sample, wherein the raw mass spectrum data (1) comprises a plurality of data points (2) with information on retention time (RT), mass-to-charge ratio measured (m/z) and intensity of the signal measured;

   consulting (104) a molecular formula database (10) which includes the theoretical mass-to-charge ratio $(m/z)^T$ of the molecular ion of a plurality of molecular formulas and ionisation adducts;

   for each data point (2) of the raw mass spectrum data (1), annotating (106) in an annotation database (12) the combinations of molecular formulas and ionisation adducts the theoretical mass-to-charge ratio $(m/z)^T$ of which corresponds to the mass-to-charge ratio (m/z) measured of said data point (2) considering a given mass error, wherein each annotation includes the retention time (RT) and the intensity of the measured signal of the data point (2);

   for each molecular formula and ionisation adduct annotated in the annotation database (12), detecting (108) regions of interest defined in a retention time range $(RT_0\text{-}RT_1)$ wherein the annotated data points meet characterisation criteria, wherein detecting (108) regions of interest comprises:

   determining (122) candidate regions (20) defined in a retention time range $(RT_{C0}\text{-}RT_{C1})$ with a minimum number of data points and/or a minimum density of data points annotated;
   characterising (124) the candidate regions (20), obtaining characterisation parameters (22); and
   selecting (128) those candidate regions (20) the characterisation parameters (22) of which meet certain characterisation criteria as regions of interest;

   generating (110) an inclusion list (14) which includes the retention time ranges $(RT_0\text{-}RT_1)$ of the detected regions of interest and the theoretical mass-to-charge ratios $(m/z)^T$ of the molecular formulas and ionisation adducts associated with each of the regions of interest;
   sending (112) the inclusion list to a mass spectrometer for the identification of compounds in the sample by means of tandem mass spectrometry; and
   performing a tandem mass spectrometry analysis using the information comprised in the inclusion list in order to identify compounds in the sample.

2. The computer-implemented method of claim 1, which comprises detecting in the raw mass spectrum data (1) isotopologues associated with the molecular formulas and/or ionisation adducts annotated, wherein the detection of isotopologues comprises:

   searching (162), in the retention time range $(RT_0\text{-}RT_1)$ of each region of interest (28), for data points (2) of the raw mass spectrum data (1) the mass-to-charge ratio measured (m/z) of which corresponds, considering a mass error, to a theoretical mass-to-charge ratio $(m/z)^T$ of an isotopologue of the molecular formula and/or ionisation adduct associated with the region of interest (28);
obtaining (164) the intensity of the measured signal of the data points found;
calculating (166) a theoretical intensity of the data points found starting from the intensity of the measured signal of the data points of the region of interest (28) corresponding to the molecular formula and/or ionisation adduct;
comparing (168) the measured intensities with the calculated theoretical intensities;
determining (170) the detection of the isotopologue based on said comparison.

3. The computer-implemented method of any of the preceding claims, wherein the characterisation (124) of the candidate regions (20) comprises calculating (132) a slope (m) of a linear regression (24) from the data points (2) annotated in the candidate regions (20);
and wherein the characterisation criteria comprise verifying (142) that the absolute value of the slope (m) calculated is greater than a threshold slope $(m_{min})$.

4. The computer-implemented method of any of the preceding claims, wherein the characterisation (124) of the candidate regions (20) comprises calculating (134, 136) an average intensity $(I_{avg})$ and/or a maximum intensity $(I_{max})$ of the measured signal from the data points (2) annotated in the candidate regions (20);
and wherein the characterisation criteria comprise verifying (144, 146) that the average intensity $(I_{avg})$ and/or the maximum intensity $(I_{max})$ calculated is greater than an average intensity $(I_{avg}^{TH})$ and/or threshold maximum intensity $(I_{max}^{TH})$.

5. The computer-implemented method of any of the preceding claims, wherein the characterisation (124) of the candidate regions (20) comprises calculating (138) an intensity range of the signal measured from the data points (2) annotated in the candidate regions (20), the intensity range being defined by a ratio between the maximum intensity and the minimum intensity in the candidate region (20);
and wherein the characterisation criteria comprise verifying (148) that the calculated intensity range is greater than a threshold intensity range.

6. The computer-implemented method of any of the preceding claims, wherein the characterisation (124) of the candidate regions (20) comprises calculating (140) a signal-to-noise ratio (SNR) between an intensity level associated with the data points (2) annotated in the candidate region (20) and an intensity level associated with the data points (2) of the raw mass spectrum data (1) located in an area surrounding (26) the candidate region (20);
and wherein the characterisation criteria comprise verifying (150) that the signal-to-noise ratio (SNR) calculated is greater than a threshold signal-to-noise ratio $(SNR^{TH})$.

7. The computer-implemented method of claim 6, wherein the area surrounding (26) the candidate region (20) is defined by a space delimited by a mass-to-charge ratio range $(m/z_{P0}\text{-}m/z_{P1})$ which includes a mass-to-charge ratio range $(m/z_{C0}\text{-}m/z_{C1})$ corresponding to the candidate region (20), and for a retention time range $(RT_{P0}\text{-}RT_{P1})$ which includes the retention time range $(RT_{C0}\text{-}RT_{C1})$ corresponding to the candidate region 20.

8. The computer-implemented method of any of the preceding claims, comprising:

   - defining a set of molecular formulas depending on the sample to be analysed;
   - defining ionisation adducts associated with the molecular formulas; and
   - generating the molecular formula database (10) including, for each molecular formula and associated ionisation adduct, the theoretical mass-to-charge ratio $(m/z)^T$.

9. The computer-implemented method of any of the preceding claims, which comprises performing a mass spectrometry analysis coupled with a separation technique applied to the sample to obtain the raw mass spectrum data (1).

10. A system for the identification of compounds in complex biological or environmental samples, **characterised in that** it comprises a control unit with data processing means configured to execute the steps of the computer-implemented method according to any of claims 1-9.

11. The system of claim 10, comprising a mass spectrometer responsible for performing a mass spectrometry analysis coupled with a separation technique on the sample in order to obtain the raw mass spectrum data (1).

12. The system of any of claims 10 to 11, comprising a mass spectrometer responsible for performing a tandem mass spectrometry analysis using the information included in the inclusion list in order to identify compounds in the sample.

13. A programme product for the identification of compounds in complex biological or environmental samples, comprising programme instructions for carrying out the computer-implemented method defined in any of claims 1-9 when the programme is executed in a processor.

14. The programme product according to claim 13, comprising at least one computer-readable storage medium which stores the programme instructions.

**Patentansprüche**

1. Computerimplementiertes Verfahren zur Identifizierung von Verbindungen in komplexen biologischen oder umweltbezogenen Proben, **dadurch gekennzeichnet, dass** es Folgendes umfasst:

   Empfangen (102) von rohen Massenspektrumsdaten (1) aus einer Massenspektrometrieanalyse, die mit einer auf eine Probe angewandten Trenntechnik gekoppelt ist, wobei die rohen Massenspektrumsdaten (1) eine Vielzahl von Datenpunkten (2) mit Informationen über die Retentionszeit (RT), das gemessene Masse-Ladung-Verhältnis (m/z) und die Intensität des gemessenen Signals umfassen;
   Abfragen (104) einer Molekularformeldatenbank (10), die das theoretische Masse-Ladung-Verhältnis $(m/z)^T$ des molekularen Ions aus einer Vielzahl von Molekularformeln und Ionisationsaddukten einschließt;
   Annotieren, (106) für jeden Datenpunkt (2) der rohen Massenspektrumsdaten (1), in einer Annotationsdatenbank (12) der Kombinationen von Molekularformeln und Ionisationsaddukten, deren theoretisches Masse-Ladung-Verhältnis $(m/z)^T$ dem gemessenen Masse-Ladung-Verhältnis (m/z) des Datenpunkts (2) unter Berücksichtigung eines gegebenen Massenfehlers entspricht, wobei jede Annotation die Retentionszeit (RT) und die Intensität des gemessenen Signals des Datenpunkts (2) einschließt;
   Detektieren (108), für jede Molekularformel und jedes Ionisationsaddukt, die in der Annotationsdatenbank (12) annotiert sind, von Regionen von Interesse, die in einem Retentionszeitbereich $(RT_0\text{-}RT_1)$ definiert sind, wobei die annotierten Datenpunkte Charakterisierungskriterien erfüllen, wobei das Detektieren (108) von Regionen von Interesse Folgendes umfasst:

      Bestimmen (122) von Kandidatenregionen (20), die in einem Retentionszeitbereich $(RT_{C0}\text{-}RT_{C1})$ definiert sind, mit einer Mindestanzahl von Datenpunkten und/oder einer Mindestdichte von annotierten Datenpunkten;
      Charakterisieren (124) der Kandidatenregionen (20), wobei Charakterisierungsparameter (22) erhalten werden; und
      Auswählen (128) derjenigen Kandidatenregionen (20), deren Charakterisierungsparameter (22) gewisse Charakterisierungskriterien erfüllen, als Regionen von Interesse;
   Generieren (110) einer Einschlussliste (14), die die Retentionszeitbereiche $(RT_0\text{-}RT_1)$ der detektierten Regionen von Interesse und die theoretischen Masse-Ladung-Verhältnisse $(m/z)^T$ der Molekularformeln und Ionisationsaddukte einschließt, die mit jeder der Regionen von Interesse assoziiert sind;
   Senden (112) der Einschlussliste an ein Massenspektrometer zur Identifizierung von Verbindungen in der Probe mittels Tandem-Massenspektrometrie; und
   Durchführen einer Tandem-Massenspektrometrieanalyse unter Verwendung der in der Einschlussliste umfassten Informationen, um Verbindungen in der Probe zu identifizieren.

2. Computerimplementiertes Verfahren nach Anspruch 1, das das Detektieren von Isotopologen in den rohen Massenspektrumsdaten (1) umfasst, die mit den annotierten Molekularformeln und/oder Ionisationsaddukten assoziiert sind, wobei das Detektieren von Isotopologen Folgendes umfasst:

   Suchen (162) im Retentionszeitbereich $(RT_0\text{-}RT_1)$ jeder Region von Interesse (28) nach Datenpunkten (2) der rohen Massenspektrumsdaten (1), deren gemessenes Masse-Ladung-Verhältnis (m/z) unter Berücksichtigung eines Massenfehlers einem theoretischen Masse-Ladung-Verhältnis $(m/z)^T$ eines Isotopologen der Molekular-formel und/oder des Ionisationsaddukts entspricht, das mit der Region von Interesse (28) assoziiert ist;
   Erhalten (164) der Intensität des gemessenen Signals der gefundenen Datenpunkte;
   Berechnen (166) einer theoretischen Intensität der gefundenen Datenpunkte, ausgehend von der Intensität des gemessenen Signals der Datenpunkte der Region von Interesse (28), die der Molekularformel und/oder dem Ionisationsaddukt entspricht;

Vergleichen (168) der gemessenen Intensitäten mit den berechneten theoretischen Intensitäten;
Bestimmen (170) der Detektion des Isotopologs basierend auf dem Vergleich.

3. Computerimplementiertes Verfahren nach einem der vorhergehenden Ansprüche, wobei die Charakterisierung (124) der Kandidatenregionen (20) das Berechnen (132) einer Steigung (m) einer linearen Regression (24) aus den in den Kandidatenregionen (20) annotierten Datenpunkten (2) umfasst; und wobei die Charakterisierungskriterien das Verifizieren (142) umfassen, dass der Absolutwert der berechneten Steigung (m) größer als eine Schwellenwertsteigung ($m_{min}$) ist.

4. Computerimplementiertes Verfahren nach einem der vorhergehenden Ansprüche, wobei die Charakterisierung (124) der Kandidatenregionen (20) das Berechnen (134, 136) einer durchschnittlichen Intensität ($I_{avg}$) und/oder einer maximalen Intensität ($I_{max}$) des gemessenen Signals aus den in den Kandidatenregionen (20) annotierten Datenpunkten (2) umfasst; und wobei die Charakterisierungskriterien das Verifizieren (144, 146) umfassen, dass die berechnete durchschnittliche Intensität ($I_{avg}$) und/oder die berechnete maximale Intensität ($I_{max}$) größer ist als eine durchschnittliche Intensität ($I_{avg}^{TH}$) und/oder eine maximale Schwellenwertintensität ($I_{max}^{TH}$).

5. Computerimplementiertes Verfahren nach einem der vorhergehenden Ansprüche, wobei die Charakterisierung (124) der Kandidatenregionen (20) das Berechnen (138) eines Intensitätsbereichs des Signals umfasst, das von den in den Kandidatenregionen (20) annotierten Datenpunkten (2) gemessen wird, wobei der Intensitätsbereich durch ein Verhältnis zwischen der maximalen Intensität und der minimalen Intensität in der Kandidatenregion (20) definiert ist; und wobei die Charakterisierungskriterien das Verifizieren (148) umfassen, dass der berechnete Intensitätsbereich größer als ein Schwellenwert-Intensitätsbereich ist.

6. Computerimplementiertes Verfahren nach einem der vorhergehenden Ansprüche, wobei die Charakterisierung (124) der Kandidatenregionen (20) das Berechnen (140) eines Signal-Rausch-Verhältnisses (SNR) zwischen einem Intensitätspegel, der mit den in der Kandidatenregion (20) annotierten Datenpunkten (2) assoziiert ist, und einem Intensitätspegel umfasst, der mit den Datenpunkten (2) der rohen Massenspektrumsdaten (1) assoziiert ist, die sich in einem die Kandidatenregion (20) umgebenden Bereich (26) befinden; und wobei die Charakterisierungskriterien das Verifizieren (150) umfassen, dass das berechnete Signal-Rausch-Verhältnis (SNR) größer ist als ein Schwellenwert-Signal-Rausch-Verhältnis ($SNR^{TH}$).

7. Computerimplementiertes Verfahren nach Anspruch 6, wobei der die Kandidatenregion (20) umgebende Bereich (26) durch einen Raum definiert ist, der durch einen Masse-Ladung-Verhältnis-Bereich ($m/z_{P0}$-$m/z_{P1}$), der einen Masse-Ladung-Verhältnis-Bereich ($m/z_{C0}$-$m/z_{C1}$) entsprechend der Kandidatenregion (20) einschließt, und durch einen Retentionszeitbereich ($RT_{P0}$-$RT_{P1}$), der den Retentionszeitbereich ($RT_{C0}$-$RT_{C1}$) entsprechend der Kandidatenregion 20 einschließt, begrenzt ist.

8. Computerimplementiertes Verfahren nach einem der vorhergehenden Ansprüche, das Folgendes umfasst:

   - Definieren eines Satzes von Molekularformeln in Abhängigkeit von der zu analysierenden Probe;
   - Definieren von Ionisationsaddukten, die mit den Molekularformeln assoziiert sind; und
   - Generieren der Molekularformeldatenbank (10), die für jede Molekularformel und jedes assoziierte Ionisationsaddukt das theoretische Masse-Ladung-Verhältnis ($m/z$)$^T$ einschließt.

9. Computerimplementiertes Verfahren nach einem der vorhergehenden Ansprüche, das das Durchführen einer Massenspektrometrieanalyse gekoppelt mit einer auf die Probe angewandten Trenntechnik umfasst, um die rohen Massenspektrumsdaten (1) zu erhalten.

10. System zur Identifizierung von Verbindungen in komplexen biologischen oder umweltbezogenen Proben, **dadurch gekennzeichnet, dass** es eine Steuereinheit mit Datenverarbeitungsmitteln umfasst, die ausgebildet sind, um die Schritte des computerimplementierten Verfahrens nach einem der Ansprüche 1-9 auszuführen.

11. System nach Anspruch 10, umfassend ein Massenspektrometer, das für das Durchführen einer Massenspektrometrieanalyse verantwortlich ist, gekoppelt mit einer Trenntechnik an der Probe, um die rohen Massenspektrumsdaten zu erhalten (1).

12. System nach einem der Ansprüche 10 bis 11, umfassend ein Massenspektrometer, das für das Durchführen einer

**EP 4 078 600 B1**

Tandem-Massenspektrometrieanalyse unter Verwendung der in der Einschlussliste eingeschlossenen Informationen verantwortlich ist, um Verbindungen in der Probe zu identifizieren.

13. Programmprodukt zur Identifizierung von Verbindungen in komplexen biologischen oder umweltbezogenen Proben, das Programmanweisungen zum Durchführen des in einem der Ansprüche 1-9 definierten computerimplementierten Verfahrens umfasst, wenn das Programm in einem Prozessor ausgeführt wird.

14. Programmprodukt nach Anspruch 13, umfassend mindestens ein computerlesbares Speichermedium, das die Programmanweisungen speichert.

## Revendications

1. Procédé mis en oeuvre par ordinateur pour l'identification de composés dans des échantillons biologiques ou environnementaux complexes, **caractérisé en ce qu'**il comprend :

   recevoir (102) des données brutes de spectre de masse (1) à partir d'une analyse de spectrométrie de masse couplée avec une technique de séparation appliquée à un échantillon, dans lequel les données brutes de spectre de masse (1) comprennent une pluralité de points de données (2) avec des informations sur le temps de rétention (RT), le rapport entre la masse et la charge mesuré (m/z) et l'intensité du signal mesuré ;
   consulter (104) une base de données de formules moléculaires (10) comprenant le rapport entre la masse et la charge théorique $(m/z)^T$ de l'ion moléculaire d'une pluralité de formules moléculaires et d'adduits d'ionisation ;
   pour chaque point de données (2) des données brutes de spectre de masse (1), annoter (106) dans une base de données d'annotation (12) les combinaisons de formules moléculaires et d'adduits d'ionisation dont le rapport entre la masse et la charge théorique $(m/z)^T$ correspond au rapport entre la masse et la charge (m/z) mesuré dudit point de données (2) en tenant compte d'une erreur de masse donnée, dans lequel chaque annotation comporte le temps de rétention (RT) et l'intensité du signal mesuré du point de données (2) ;
   pour chaque formule moléculaire et adduit d'ionisation annoté dans la base de données d'annotation (12), détecter (108) des régions d'intérêt définies dans une plage de temps de rétention $(RT_0-RT_1)$ dans laquelle les points de données annotés satisfont des critères de caractérisation, dans lequel la détection (108) de régions d'intérêt comprend :

      déterminer (122) des régions candidates (20) définies dans une plage de temps de rétention $(RT_{C0}-RT_{C1})$ avec un nombre minimal de points de données et/ou une densité minimale de points de données annotés ;
      caractériser (124) les régions candidates (20), en obtenant des paramètres de caractérisation (22) ; et
      sélectionner (128) les régions candidates (20) dont les paramètres de caractérisation (22) satisfont certains critères de caractérisation comme régions d'intérêt ;

   générer (110) une liste d'inclusion (14) comportant les plages de temps de rétention $(RT_0-RT_1)$ des régions d'intérêt détectées et les rapports entre la masse et la charge théoriques $(m/z)^T$ des formules moléculaires et d'adduits d'ionisation associés avec chacune des régions d'intérêt ;
   envoyer (112) la liste d'inclusion à un spectromètre de masse pour l'identification de composés dans l'échantillon par le biais de spectrométrie de masse en tandem ; et
   effectuer une analyse de spectrométrie de masse en tandem en utilisant les informations comprises dans la liste d'inclusion afin d'identifier des composés dans l'échantillon.

2. Procédé mis en oeuvre par ordinateur selon la revendication 1, comprenant la détection, dans les données brutes de spectre de masse (1), d'isotopologues associés avec les formules moléculaires et/ou les adduits d'ionisation annotés, dans lequel la détection d'isotopologues comprend :

   rechercher (162), dans la plage de temps de rétention $(RT_0-RT_1)$ de chaque région d'intérêt (28), des points de données (2) des données brutes de spectre de masse (1) dont le rapport entre la masse et la charge mesuré (m/z) correspond, en tenant compte d'une erreur de mass, à un rapport entre la masse et la charge théorique $(m/z)^T$ d'un isotopologue de la formule moléculaire et/ou l'adduit d'ionisation associé avec la région d'intérêt (28) ;
   obtenir (164) l'intensité du signal mesuré des points de données trouvés ;
   calculer (166) une intensité théorique des points de données trouvés à partir de l'intensité du signal mesuré des points de données de la région d'intérêt (28) correspondant à la formule moléculaire et/ou l'adduit d'ionisation ;

comparer (168) les intensités mesurées avec les intensités théoriques calculées ;

déterminer (170) la détection de l'isotopologue sur la base de ladite comparaison.

3. Procédé mis en oeuvre par ordinateur selon l'une quelconque des revendications précédentes, dans lequel la caractérisation (124) des régions candidates (20) comprend le calcul (132) d'une pente (m) d'une régression linéaire (24) à partir des points de données (2) annotés dans les régions candidates (20) ;

et dans lequel les critères de caractérisation comprennent la vérification (142) que la valeur absolue de la pente (m) calculée est supérieure à une pente seuil ($m_{min}$).

4. Procédé mis en oeuvre par ordinateur selon l'une quelconque des revendications précédentes, dans lequel la caractérisation (124) des régions candidates (20) comprend le calcul (134, 136) d'une intensité moyenne ($I_{avg}$) et/ou d'une intensité maximale ($I_{max}$) du signal mesuré à partir des points de données (2) annotés dans les régions candidates (20) ;

et dans lequel les critères de caractérisation comprennent la vérification (144, 146) que l'intensité moyenne ($I_{avg}$) et/ou l'intensité maximale ($I_{max}$) calculée est supérieure à une intensité moyenne ($I_{avg}^{TH}$) et/ou une intensité maximale seuil ($I_{max}^{TH}$).

5. Procédé mis en oeuvre par ordinateur selon l'une quelconque des revendications précédentes, dans lequel la caractérisation (124) des régions candidates (20) comprend le calcul (138) d'une plage d'intensité du signal mesuré à partir des points de données (2) annotés dans les régions candidates (20), la plage d'intensité étant définie par un rapport entre l'intensité maximale et l'intensité minimale dans la région candidate (20) ;

et dans lequel les critères de caractérisation comprennent la vérification (148) que la plage d'intensité calculée est supérieure à une plage d'intensité range seuil.

6. Procédé mis en oeuvre par ordinateur selon l'une quelconque des revendications précédentes, dans lequel la caractérisation (124) des régions candidates (20) comprend le calcul (140) d'un rapport signal/bruit (SNR) entre un niveau d'intensité associé avec les points de données (2) annotés dans la région candidate (20) et un niveau d'intensité associé avec les points de données (2) des données brutes de spectre de masse (1) situés dans une zone (26) entourant la région candidate (20) ;

et dans lequel les critères de caractérisation comprennent la vérification (150) que le rapport signal/bruit (SNR) calculé est supérieur à un rapport signal/bruit seuil ($SNR^{TH}$).

7. Procédé mis en oeuvre par ordinateur selon la revendication 6, dans lequel la zone (26) entourant la région candidate (20) est définie par un espace délimité par un rapport entre la masse et la charge range ($m/z_{P0}$-$m/z_{P1}$) qui comporte une plage de rapport entre la masse et la charge ($m/z_{C0}$-$m/z_{C1}$) correspondant à la région candidate (20), et pour une plage de temps de rétention ($RT_{P0}$-$RT_{P1}$) qui comporte la plage de temps de rétention ($RT_{C0}$-$RT_{C1}$) correspondant à la région candidate.

8. Procédé mis en oeuvre par ordinateur selon l'une quelconque des revendications précédentes, comprenant :

- définir un ensemble de formules moléculaires en fonction de l'échantillon à analyser ;
- définir des adduits d'ionisation associés avec les formules moléculaires ; et
- générer la base de données de formules moléculaires (10) comportant, pour chaque formule moléculaire et adduit d'ionisation associé, le rapport entre la masse et la charge théorique ($m/z)^T$.

9. Procédé mis en oeuvre par ordinateur selon l'une quelconque des revendications précédentes, comprenant la réalisation d'une analyse de spectrométrie de masse couplée avec une technique de séparation appliquée à l'échantillon pour obtenir les données brutes de spectre de masse (1).

10. Système pour l'identification de composés dans des échantillons biologiques ou environnementaux complexes, **caractérisé en ce qu'**il comprend une unité de commande avec des moyens de traitement de données configurés pour exécuter les étapes du procédé mis en oeuvre par ordinateur selon l'une quelconque des revendications 1-9.

11. Système selon la revendication 10, comprenant un spectromètre de masse responsable de réaliser une analyse de spectrométrie de masse couplée avec une technique de séparation sur l'échantillon afin d'obtenir les données brutes de spectre de masse (1).

12. Système selon l'une quelconque des revendications 10 à 11, comprenant un spectromètre de masse responsable de réaliser une analyse de spectrométrie de masse en tandem en utilisant les informations comprises dans la liste d'inclusion afin d'identifier des composés dans l'échantillon.

13. Produit de programme pour l'identification de composés dans des échantillons biologiques ou environnementaux complexes, comprenant des instructions de programme pour mettre en oeuvre le procédé mis en oeuvre par ordinateur selon l'une quelconque des revendications 1-9 lorsque le programme est exécuté dans un processeur.

14. Produit de programme selon la revendication 13, comprenant au moins un moyen de stockage lisible par ordinateur qui stocke les instructions de programme.

Fig. 1

EP 4 078 600 B1

Fig. 2A

Fig. 2B

EP 4 078 600 B1

Fig. 3A

Fig. 3C

Fig. 3B

ATP

Fig. 4A

SAM

Fig. 4B

Fig. 5

Fig. 6A

EP 4 078 600 B1

Fig. 6B

Fig. 7

C21H27NO4+NH4

Fig. 8

EP 4 078 600 B1

CHARACTERISING CANDIDATE REGION    124

132 CALCULATING SLOPE

134 CALCULATING AVERAGE INTENSITY

136 CALCULATING MAXIMUM INTENSITY

138 CALCULATING INTENSITY RANGE

140 CALCULATING SIGNAL-TO-NOISE RATIO

$m$ 22

$I_{Avg}$ 22

$I_{max}$ 22

$\log (I_{max}/I_{min})$ 22

$SNR$ 22

126

142 $|m| > m_{min}$

144 $I_{Avg} > I_{Avg}^{TH}$

146 $I_{max} > I_{max}^{TH}$

148 $\log (I_{max}/I_{min}) > \log (I_{max}/I_{min})^{TH}$

150 $SNR > SNR^{TH}$

128 SELECTING REGIONS OF INTEREST

Fig. 9

Fig. 10

120

162
SEARCHING IN ($RT_0$-$RT_1$) FOR DATA POINTS OF THE MASS SPECTRUM WITH M/Z CORRESPONDING TO AN ISOTOPOLOGUE

164
OBTAINING MEASURED INTENSITY OF THE DATA POINTS FOUND

166
CALCULATING THEORETICAL INTENSITY FROM THE DATA POINTS FOUND

168
COMPARING MEASURED INTENSITIES WITH THEORETICAL INTENSITIES

170
DETERMINING THE DETECTION OF THE ISOTOPOLOGUE

Fig. 11

Fig. 12A

# Resolution 60000 (i.e.QTOF)

Fig. 12B

Fig. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017047209 A1

**Non-patent literature cited in the description**

- **ALEXANDRA ZERCK et al.** Optimal precursor ion selection for LC-MALDI MS/MS. *BMC Bioinformatics,* February 2013
- **SCHMIDT ALECANDER et al.** An integrated, directed mass spectrometric approach for in-depth characterization of complex peptide mixtures. *Molecular & Cellular Proteomics,* October 2007
- **NASH WILLIAM et al.** From mass to metabolite in human untargeted metabolomics: Recent advances in annotation of metabolites applying liquid chromatography-mass spectrometry data. *Trac Trends in Analytical Chemistry,* November 2018
- **ZUBAREV et al.** Orbitrap Mass Spectrometry. *Analytical Chemistry,* 2013, vol. 85 (11), 5288-5296